(19) Europäisches Patentamt
European Patent Office
Office européen des brevets

(11) Publication number: **0 136 382**
**B1**

# (12) EUROPEAN PATENT SPECIFICATION

(45) Date of publication of patent specification: **08.04.87**

(51) Int. Cl.[4]: **C 07 C 119/18**, C 07 C 125/08

(21) Application number: **83306029.6**

(22) Date of filing: **05.10.83**

(54) **Process for preparing dialkyl propanediimidate dihydrohalides.**

(43) Date of publication of application:
**10.04.85 Bulletin 85/15**

(45) Publication of the grant of the patent:
**08.04.87 Bulletin 87/15**

(84) Designated Contracting States:
**AT BE CH DE FR GB IT LI LU NL SE**

(56) References cited:
**US-A-4 310 470**

**CHEMICAL ABSTRACTS, vol. 97, no. 21, Nov 22, 1982, p. 757, no. 181647n, Columbus, Ohio (US); P.L.BARTLETT: "Use of a chlorofluorocarbon as a reaction solvent" Spec. Chem. 1981, 1(4), 31-3, 36-7**

(73) Proprietor: **E.I. DU PONT DE NEMOURS AND COMPANY**
**1007 Market Street**
**Wilmington Delaware 19898 (US)**

(72) Inventor: **Gramm, Jeffrey Scott**
**10 Oxford Court**
**Wilmington, DE 19805 (US)**

(74) Representative: **Hildyard, Edward Martin et al**
**Frank B. Dehn & Co. European Patent Attorneys**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 136 382 B1

## Description

Background of the Invention

This invention relates to a process for preparing certain dialkyl propanediimidate dihydrohalides.

McElvain and Schroeder, JACS 71, 43 (1949), disclose the preparation of dimethyl and diethyl propanediimidate dihydrochloride by treating malononitrile and the corresponding alcohol with a large excess of hydrogen chloride using a chloroform-dioxane mixture and dioxane, respectively, as solvents. The process is disclosed to give high yields, but a reaction time of about 24 hours is required.

U.S. Patent No. 4,310,470, issued on January 12, 1982 to Adams, discloses an improved process for preparing a dialkyl propanediimidate dihydrohalide by reacting malononitrile, an alcohol and hydrogen halide in an alkyl acetate solvent.

It is also known (Spec. Chem. 1981, 1(4), 31—3, 36—7) to use 1,1,2-trichloro-1,2,2-trifluoroethane as a reaction solvent in such processes as sulfonation, Friedel-Crafts alkylation and heterogeneous polymerisations. However, it has never been proposed to use this solvent to prepare dialkyl propanediimidate dihydrohalides.

Imido ester hydrohalides are well-known compounds. They are useful as chemical intermediates for other chemical compounds such as amidine hydrochlorides or as intermediates for herbicides as described in U.S. Patents 4,287,343 and 4,299,960. Improvements in the process for preparing these imido ester hydrohalides are increasingly desirable and are constantly being sought. Furthermore, an improved process which provides greater ease of operation and shorter reaction times offers even greater attractions.

Summary of the Invention

An improved process for preparing dialkyl propanediimidate dihydrohalides of the formula:

$$CH_2(\overset{OR}{\overset{|}{C}}{=}NH \cdot HX)_2,$$

where

R=alkyl of 1 to 3 carbons, and X is chlorine or bromine has now been found. This new process involves contacting malononitrile, an alcohol ROH, where R is as defined above, and anhydrous hydrogen halide HX, where X is chlorine or bromine, in the presence of a chlorofluorocarbon solvent having a boiling point in the range of about −45 to 100°C at atmospheric pressure.

This new process offers a number of advantages over the best previously known process, that described in U.S. Patent 4,310,470 to Adams. Not only is the rate of reaction in this new process faster than that in the Adams, but the process can be run with greater ease due to the fact that the dialkyl propanediimidate dihydrohalide need not be isolated before use in subsequent reactions, and that the chlorofluorocarbon solvent is nonflammable, is easily recycled, and gives less corrosive reaction mixtures when mixed with hydrogen halides than does methyl acetate. Additionally, in the preferred embodiment of the Adams process, the hydrohalic acid is used in an amount sufficient to saturate the solvent. In the preferred embodiment of the new process described and claimed herein, the hydrohalic acid is used in an excess of only thirty percent over the stoichiometric amount, thus conserving material and reducing waste disposal problems.

Detailed Description of the Invention

The chlorofluorocarbon solvent utilized in the improved process of this invention has a boiling point in the range of about −45 to 100°C, preferably 10 to 100°C at atmospheric pressure. The optimal solvent to use in the process will depend on the temperature and pressure under which the reaction is run. When higher pressures are used to speed up reaction times, solvents with lower boiling points will be desired. A number of suitable chlorofluorocarbons, all methane and ethane derivatives, are commercially available. See, for example, "'Freon' Product Information," Bulletin B-2, a technical bulletin of E. I. du Pont de Nemours and Co., to which the readers is referred for further information and for a listing of representative chlorofluorocarbons. Suitable chlorofluorocarbons include but are not limited to the following: chlorodifluoromethane, chlorotetrafluoroethane, dichlorodifluoromethane, dichlorofluoromethane, 1,1,2-trichloro-1,2,2-trifluoroethane, 1,1,2,2-tetrachloro-1,2-difluoroethane, 1,2-dichloro-1,1,2,2-tetrafluoroethane, dichlorofluoromethane and trichlorofluoromethane. Mixtures of various chlorofluorocarbons can also be used. For example, commercially available azeotropic mixtures of dichlorodifluoromethane/1,1-difluoroethane and of chlorodifluoromethane/chlorotetrafluoroethane could be used as solvents in the process of this invention. The preferred chlorofluorocarbon for use as solvent in the claimed process is 1,1,2-trichloro-1,2,2-trifluoroethane, available from the Du Pont Company under the trademark Freon TF®.

In the preferred practice of this invention, about 2 to 3 molar equivalents, based on malononitrile, of alcohol are used. Preferably about 2 to 2.2, and more preferably about 2.2, molar equivalents of alcohol are

used. About 2 to 4 molar equivalents, also based on malononitrile, of hydrogen halide are used. Preferably about 2.5 to 4, and more preferably about 2.6, molar equivalents of hydrogen halide are used.

The malononitrile concentration in the chlorofluorocarbon solvent can vary e.g. from about 1 to 20 weight %. A concentration of about 5—15 weight % is preferred and 5—10 weight % is more preferred.

The process is preferably run at a temperature in the range of about 0 to 40°C, more preferably about 20 to 30°C. Pressure is not critical, and the reaction can be run under atmospheric pressure or higher pressure, the advantage of higher pressure being that reaction times can be shortened. The reaction is preferably run under pressure in the range of about 0 to 100 psig (101.3 to 790.3 kPa), more preferably about 10 to 100 psig (170.2 to 790.3 kPa). When X=Cl, it is, of course, practical to run the reaction under HCl pressure, and when X=Br it is practical to run the reaction under HBr pressure. All pressures defined in metric units are absolute pressures.

The order in which the reactants are contacted is not critical except that the malononitrile and the hydrogen halide are preferably not contacted in the absence of the alcohol. The reaction mixture should be agitated to ensure that the malononitrile is thoroughly dispersed in the solvent.

The process of this invention is further illustrated by the following examples, in which temperatures are in degrees centigrade and parts are by weight unless otherwise specified. These examples are provided to illustrate the process of this invention.

Example 1

Preparation of Dimethyl Propanediimidate Dihydrochloride

A mixture of 58 parts of malononitrile, 63 parts of methanol, and 783 parts of 1,1,2-trichloro-1,2,2-trifluoroethane (Freon TF®) was stirred in a 1-L pressure vessel while anhydrous hydrogen chloride was introduced at a pressure of 20 psig (137.9 kPa). The mixture was stirred and held at 23—27° with cooling and the HCl pressure was maintained at 20 psig (239.1 kPa) until 83 parts of HCl were introduced (2.5 hours). HCl feed was then discontinued, and the reaction mass was stirred another 2 hours at 23—27°. The resulting slurry was filtered, and the solid product was washed with Freon TF® solvent and dried at room temperature to give 171 parts (96% yield based on malononitrile charged) of the title compound, which was identified by comparison with material produced as described in U.S. Patent 4,310,470.

Example 2

Preparation of Dimethyl Propanediimidate Dihydrochloride and Its Use in a Subsequent Reaction Without Isolation

An important advantage of chlorofluorocarbon solvents over those previously used in processes for preparing dialkyl propanediimidate dihydrohalides, namely, the ability to use the crude reaction product in subsequent reactions with workup, is illustrated as follows.

A 1-L pressure vessel was charged as in Example 1 and pressured to 24.5 psig (270.1 kPa) with anhydrous hydrogen chloride gas. HCl was introduced as required to maintain this pressure, and the mixture was cooled and stirred at 25° until 83 parts of HCl had been added (3 hours). HCl feed was then discontinued, and the reaction mass was stirred overnight at 25°.

The entire reaction mass was then transferred gradually to a 2-L vessel containing a well-stirred mixture of 80 parts of 50% aqueous cyanamide and 500 parts of water. During this addition, the reaction mixture was held below 10° by external cooling, and 50% aqueous sodium hydroxide was added as required to hold the pH between 5 and 7. The resulting slurry was warmed to room temperature, stirred for two hours, and filtered. The solid product was washed with water and dried under a stream of nitrogen at room temperature to give 76.5 parts (56% based on malononitrile) of methyl 3-amino-3-methoxy-N-cyano-2-propenimidate. The identity of this material was established by comparison with material produced by the procedure of U.S. Patent 4,235,802.

This product was of sufficient purity for conversion to 2-amino-4,6-dimethoxypyrimidine as described in U.S. Patent 4,299,960.

**Claims**

1. A process for preparing a dialkyl propanediimidate dihydrohalide of the formula:

$$\begin{matrix} & OR \\ & | \\ CH_2( & C=NH\cdot HX)_2, \end{matrix}$$

where

R is alkyl of 1 to 3 carbon atoms, and

X is chlorine or bromine,

by reacting malononitrile, an alcohol ROH and anhydrous hydrogen halide HX, characterised in that the reaction is conducted in a chlorofluorocarbon solvent having a boiling point in the range of about −45 to 100°C.

2. The process of Claim 1 where the solvent has a boiling point in the range of about 10 to 100°C.

3. The process of Claim 2 where the solvent is 1,1,2-trichloro-1,2,2-trifluoroethane.

4. The process of Claims 1 to 3 where the reaction is conducted under an absolute pressure of about 170.2 to 790.3 kPa.

5. The process of any of the preceding Claims where the reaction is conducted at a temperature in the range of about 0 to 40°C.

6. The process of Claim 5 where the reaction is conducted at a temperature of about 20 to 30°C.

7. The process of any of the preceding Claims where about 2 to 3 moles of alcohol ROH are present per mole of malononitrile.

8. The process of Claim 7 where about 2.2 moles of alcohol ROH are present per mole of malononitrile.

9. The process of any of the preceding Claims where about 2 to 4 moles of hydrogen halide HX are present per mole of malononitrile.

10. The process of Claim 9 where about 2.6 moles of hydrogen halide HX are present per mole of malononitrile.

11. The process of any of the preceding Claims where the concentration of the malononitrile in the chlorofluorocarbon solvent is in the range of about 5 to 10 weight percent.

12. The process of any of the preceding Claims where $R=CH_3$ and $X=Cl$.

13. The process of Claim 1 where dimethyl propanediimidate dihydrochloride is prepared by contacting malononitrile with about 2.2 moles of methanol and about 2.6 moles of anhydrous hydrogen chloride, per mole of malononitrile, in 1,1,2-trichloro-1,2,2-trifluoroethane solvent, at a temperature in the range of about 20 to 30°C and under an HCl absolute pressure of about 170.2 to 790.3 kPa.

14. A process for the production of an ($C_{1-3}$ alkyl) 3-amino-3-($C_{1-3}$ alkoxy)-N-cyano-2-propaneimidate which comprises reacting the dialkyl propanediimidate dihydrohalide produced by the process of any of the preceding claims, without isolation from said chlorofluorocarbon solvent, with cyanamide and a base.

**Patentansprüche**

1. Verfahren zur Herstellung eines Dialkylpropandiimidatdihydrohalogenids der Formel

OR
|
$CH_2(C=NH \cdot HX)_2$,

worin R Alkyl mit 1 bis 3 Kohlenstoffatomen ist und
X Chlor oder Brom ist,
durch Reagieren von Malononitril, einem Alkohol ROH und wasserfreiem Halogenwasserstoff HX, dadurch gekennzeichnet, dass die Reaktion in einem Chlorfluorkohlenstofflösungsmittel mit einem Siedepunkt im Bereich von etwa −45 bis 100°C durchgeführt wird.

2. Verfahren nach Anspruch 1, worin das Lösungsmittel einen Siedepunkt im Bereich von etwa 10 bis 100°C aufweist.

3. Verfahren nach Anspruch 2, worin das Lösungsmittel 1,1,2-Trichlor-1,2,2-trifluorethan ist.

4. Verfahren nach einem der Ansprüche 1 bis 3, worin die Reaktion unter einem absoluten Druck von 170,2 bis 790,3 kPa durchgeführt wird.

5. Verfahren nach einem der vorstehenden Ansprüche, worin die Reaktion bei einer Temperatur im Bereich von 0 bis 40°C durchgeführt wird.

6. Verfahren nach Anspruch 5, worin die Reaktion bei einer Temperatur von etwa 20 bis 30°C durchgeführt wird.

7. Verfahren nach einem der vorstehenden Ansprüche, worin etwa 2 bis 3 Mol Alkohol ROH pro Mol Malononitril vorhanden sind.

8. Verfahren nach Anspruch 7, worin etwa 2,2 Mol Alkohol ROH pro Mol Malononitril vorhanden sind.

9. Verfahren nach einem der vorstehenden Ansprüche, worin etwa 2 bis 4 Mol Halogenwasserstoff HX pro Mol Malononitril vorhanden sind.

10. Verfahren nach Anspruch 9, worin etwa 2,6 Mol Halogenwasserstoff HX pro Mol Malononitril vorhanden sind.

11. Verfahren nach einem der vorstehenden Ansprüche, worin die Konzentration an Malononitril im Chlorfluorkohlenstofflösungsmittel im Bereich von etwa 5 bis 10 Gew.-% liegt.

12. Verfahren nach einem der vorstehenden Ansprüche, worin $R=CH_3$ und $X=Cl$.

13. Verfahren nach Anspruch 1, worin Dimethylpropandiimidatdihydrochlorid durch Inkontaktbringen von Malononitril mit etwa 2,2 Mol Methanol und 2,6 Mol wasserfreiem Chlorwasserstoff pro Mol Malononitril in 1,1,2-Trichlor-1,2,2-trifluorethanlösungsmittel bei einer Temperatur im Bereich von etwa 20 bis 30°C und unter einem absoluten HCl-Druck von etwa 170,2 bis 790,3 kPa hergestellt wird.

14. Verfahren zur Herstellung eines ($C_{1-3}$-Alkyl)-3-amino-3-($C_{1-3}$-alkoxy)-N-cyano-2-propenimidats durch Reagieren des nach dem Verfahren nach einem der vorstehenden Ansprüche hergestellten Dialkylpropandiimidatdihydrohalogenids ohne Isolierung aus dem Chlorfluorkohlenstofflösungsmittel mit Cyanamid und einer Base.

**Revendications**

1. Un procédé pour préparer un dihalogènhydrate de propanediimidate de dialkyle de la formule:

$$\begin{array}{c} \quad\;\; OR \\ \quad\;\; | \\ CH_2(C{=}NH.HX)_2 \end{array}$$

où

R est un groupe alkyle de 1 à 3 atomes de carbone, et

X est le chlore ou le brome,

en faisant réagir du malononitrile, un alcool ROH et un halogénure d'hydrogène anhydre HX, caractérisé en ce que l'on conduit la réaction dans un solvant de type chlorofluorocarbure ayant un point d'ébullition dans l'intervalle d'environ −45 à 100°C.

2. Le procédé de la revendication 1, où le solvant a un point d'ébullition dans l'intervalle d'environ 10 à 100°C.

3. Le procédé de la revendication 2, où le solvant est le 1,1,2-trichloro-1,2,2-trifluoroéthane.

4. Le procédé de l'une quelconque des revendications 1 à 3, où l'on conduit la réaction sous une pression absolue d'environ 170,2 à 790,3 kPa.

5. Le procédé de l'une quelconque des revendications précédentes, où l'on conduit la réaction à une température dans l'intervalle d'environ 0 à 40°C.

6. Le procédé de la revendication 5, où l'on conduit la réaction à une température d'environ 20 à 30°C.

7. Le procédé de l'une quelconque des revendications précédentes, où environ 2 à 3 moles d'alcool ROH sont présentes par mole de malononitrile.

8. Le procédé de la revendication 7, où environ 2,2 moles d'alcool ROH sont présentes par mole de malononitrile.

9. Le procédé de l'une quelconque des revendications précédentes, où environ 2 à 4 moles d'halogénure d'hydrogène HX sont présentes par mole de malononitrile.

10. Le procédé de la revendication 9, où environ 2,6 moles d'halogénure d'hydrogène HX sont présentes par mole de malononitrile.

11. Le procédé de l'une quelconque des revendications précédentes, où la concentration du malononitrile dans le solvant de type chlorofluorocarbure est dans le gamme d'environ 5 à 10% en poids.

12. Le procédé de l'une quelconque des revendications précédentes, où $R{=}CH_3$ et $X{=}Cl$.

13. Le procédé de la revendication 1, où l'on prépare du dichlorhydrate de propanediimidate de diméthyle en mettant en contact du malononitrile avec environ 2,2 moles de méthanol et environ 2,6 moles de chlorure d'hydrogène anhydre par mole de malononitrile, dans du 1,1,2-trichloro-1,2,2-trifluoroéthane comme solvant, à une température dans l'intervalle d'environ 20 à 30°C et sous une pression absolue de HCl d'environ 170,2 à 790,3 kPa.

14. Un procédé pour la préparation d'un 3-amino-3-(alcoxy en $C_1$ à $_3$)-N-cyano-2-propénimidate d'alkyle en $C_1$ à $_3$, qui consiste à faire réagir le dihalogénhydrate de propanediimidate de dialkyle préparé par le procédé de l'une quelconque des revendications précédentes, sans l'isoler du solvant de type chlorofluorocarbure, avec du cyanamide et une base.